# EUROPEAN PATENT APPLICATION

(11) **EP 4 124 303 A1**
(43) Date of publication of application: **01.02.2023**
(21) Application number: 22386044.6
(22) Date of filing: 05.07.2022
(51) Int. Cl.: A61B 17/04, A61B 17/06

(54) **SELF-LOCKING SURGICAL SUTURE**

(30) Priority: 26.07.2021 GR 20210100522
(71) Applicant: Gialamas, Aggelos, Kalamata (GR)
(72) Inventor: Gialamas, Aggelos, Kalamata (GR)

(57) **Abstract**

The present invention relates to self-locking surgical sutures, which are used in surgical operations or in organ injuries, for the restoration of skin and organ damages.

The sutures according to the present invention ensure safer application of sutures on various parts of the body and a much easier mounting on difficult parts.

Figure 1 shows the suture (1), according to the present invention, in side view, where the parts with toothings (2) and (3) and the needles (4) at the ends are shown. The toothings (2) and (3) extend over the one half length of the upper part and one half length of the lower part and have an opposite direction and opposite inclination.

Figure 2 shows a side view of the suture (1) according to the present invention as engaged. In Figure 2, the suture (1) is shown as engaged during application, the securing ring (7) being mounted and the ends (8) with the incorporated needles (4) being cut-off.

## Description

The present invention relates to self-locking surgical sutures, which are used in surgical operations or in organ injuries for the restoration of skin and organ damages.

Surgical sutures have been known since surgery was developed. Sutures are used in operations for the restoration of incisions in the skin and organs, in order to restore the continuity of the tissues.

Sutures are classified as absorbable and non-absorbable by the organism. The manufacturing materials of the sutures include non-absorbable silk, non-absorbable polyamide, non-absorbable polypropylene, absorbable synthetic material, non-absorbable polyester and non-absorbable stainless steel wire.

Sutures are manufactured so that they can safely hold the parts being connected and remain at the intended position for as long time considered necessary for the healing of the damage and so that they present high mechanical strength.

During their use at certain parts of the body, due to special conditions or due to enhanced requirements against stresses, problems arise as regards their locking and fixation, thereby failing to ensure the required support on the parts being connected, and thus frequently a new operation and positioning of new sutures are required, which is very inconvenient for the patients.

Advantage of the present invention is that the proposed suture is manufactured with toothings on the surface over one half length on each side and the toothings are opposite, so that the suture locks upon its application, making its release due to stresses impossible. By using a ring through which the two ends of the suture pass when the suture is mounted at its final position, stable and permanent positioning is ensured.

According to the present invention, it is desirable that the ends of the sutures have incorporated needles which are removed after their application.

A preferred embodiment of the present invention is described hereinafter by means of figures.
Figure 1 shows the proposed suture in side view.
Figure 2 shows the proposed suture as engaged.
Figure 3 shows a sectional view of the proposed suture, as engaged, having a circular cross-section.
Figure 4 shows a sectional view of a rectangular cross-section of the proposed suture as engaged.

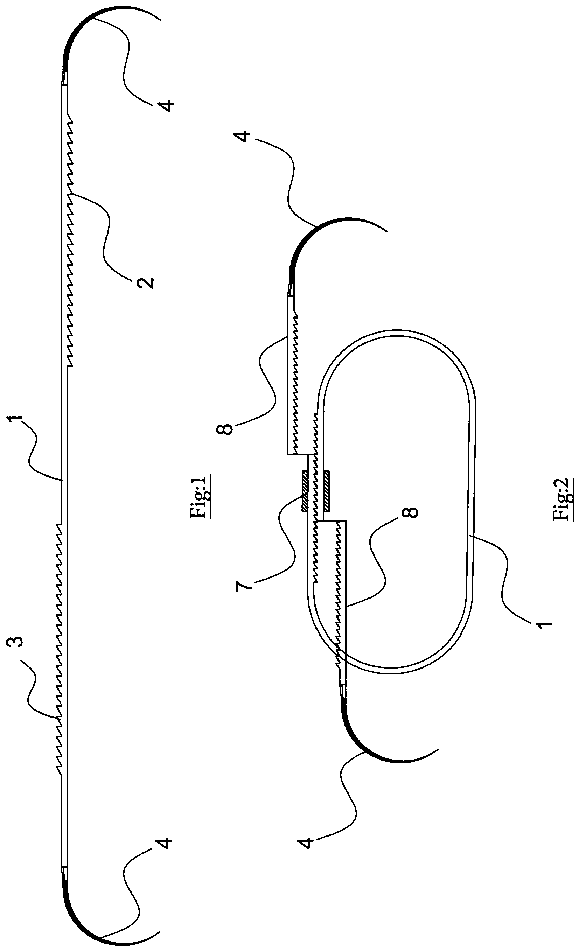

Figure 1 shows the suture (1) according to the present invention in side view, where the parts with toothings (2) and (3) and the needles (4) at the ends are shown. The toothings (2) and (3) extend over one half length of the upper part and one half length of the lower part of the suture and have an opposite direction and opposite inclination.

Figure 2 shows a side view of the suture (1) according to the present invention as engaged, where the securing ring (7) is positioned, and the ends (8) are shown cut-off, the ends being removed after the application of the suture at the final position.

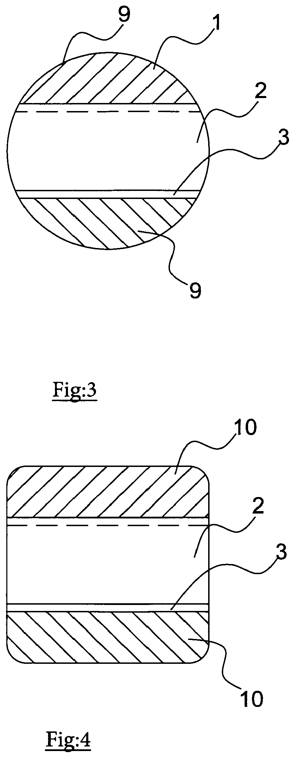

Figure 3 shows a sectional view of the suture according to the present invention, having a circular cross-section (9) when engaged, the toothings (2) and (3) being apparent.

Figure 4 shows a sectional view of the suture according to the present invention, having a rectangular cross-section (10) when engaged, the toothings (2) and (3) being apparent.

The suture (1) according to the present invention is positioned by means of the needles (4) which are incorporated at the ends. When the needles pass through the parts to be sutured, the toothings (2) and (3) are engaged and then the securing ring (7) is mounted.

After the suture becomes locked, the excess parts (8) are cut off. The ring (7) prevents disconnection of the engaged toothings and disassembly of the sutures.

The manufacturing materials of the sutures according to the present invention include polypropylene, polyamide, polyester and other synthetic non-absorbable materials compatible with the tissues of the human body.

The securing ring is manufactured from the same materials as those of the sutures or from stainless steel suitable for use in the human body.

The size of the sutures may vary and depends on the requirements of the individual use.

The toothings are not limited to those of the accompanying figures, but may be varied so that they serve the various uses, and the form of the teeth may be triangular, trapezoidal, circular or even the form of teeth may be absent and the engaged surfaces may be rough and present a high friction resistance to prevent slip.

## Claims

1. Self-locking surgical suture (1) consisting of a main body on which opposite toothings (2) and (3) are arranged which during use are engagable and securable by a ring (7), wherein at the ends of the suture needles (4) are mounted which after locking of the suture are removed along with excess parts (8), wherein the manufacturing materials of the sutures include polypropylene, polyamide, polyester and other synthetic non-absorbable or absorbable materials biocompatible with the tissues of the human body.

2. Self-locking surgical suture (1) according to claim 1 wherein the main body has a rectangular cross-section, the ring (7) not allowing disconnection of the engaged toothings and disassembly of the suture, the ring (7) having a cross-section corresponding to that of the suture.

3. Self-locking surgical suture (1) according to claim 1 wherein the main body has a semi-circular or circular cross-section, the ring (7) not allowing disconnection of the engaged toothings and disassembly of the suture, the ring (7) having a cross-section corresponding to that of the suture.
